# EUROPEAN PATENT SPECIFICATION

(11) **EP 3 818 954 B1**
(45) Date of publication and mention of the grant of the patent: **19.01.2022**
(21) Application number: 19207872.3
(22) Date of filing: 08.11.2019
(51) Int. Cl.: A61B 18/24, A61B 18/26, A61B 1/01, A61M 25/09

(54) **GUIDE WIRE FOR ENDOSCOPIC TREATMENTS, KIT COMPRISING A GUIDE WIRE AND AN ADAPTER AND MONITORING SYSTEM**
FÜHRUNGSDRAHT FÜR ENDOSKOPISCHE BEHANDLUNGEN, KIT MIT EINEM FÜHRUNGSDRAHT UND EINEM ADAPTER UND ÜBERWACHUNGSSYSTEM
FIL DE GUIDAGE POUR TRAITEMENTS ENDOSCOPIQUES, KIT COMPRENANT UN FIL DE GUIDAGE ET UN ADAPTATEUR ET SYSTÈME DE SURVEILLANCE

(43) Date of publication of application: 12.05.2021
(73) Proprietor: Ferton Holding S.A., 2800 Delémont (CH)
(72) Inventor: LANÇON, Etienne, 01210 Ferney-Voltaire (FR); BLONDEAU, Jerôme, 74350 Menthonnex en Bornes (FR); GATINEAU, Vincent, 74160 St. Julien en Genevois (FR)
(74) Representative: Müller Schupfner & Partner Patent- und Rechtsanwaltspartnerschaft mbB

(56) References cited:
- EP-B1- 1 012 912
- US-A1- 2009 192 412
- US-A1- 2018 093 078

## Description

The present invention concerns a guide wire for endoscopic treatments and a kit comprising a guide wire and an adapter, a monitoring system.

Guide wires for endoscopic treatments are well known from the state of the art. For example, the documents US 2017 0 325 673 A1, US 9,782,129 B2, US 2013 / 0 218 032 A1, EP 1 310 215 A1, EP 1 012 912 B1 and EP 1 849 409 A1 disclose such guide wires.

Typically, guide wires are part of an endoscopic system for gaining visual insight to an inner region of a human body. In particular, it is provided to introduce the guide wire into a tube-like anatomic structure, such as an ureters for instance, and to move a distal section of the guide wire to a treatment zone, such as a certain region in a kidney. In general, the guide wire has a flexible spring element for establishing a desired flexibility for realizing a necessary manoeuvrability while passing the tortuous tube-like anatomy. Further, the distal end of the guide wire is placed inside the kidney where the operation takes place. However, it is preferable that the guide wire is placed in a zone aside from the treatment zone where the lithotripsy devices will be fragmenting the stone and could damage the guide wire and the sensor. For keeping traumatization to tissues as low as possible, the distal tip of the guide wire is dimensioned comparably thin.

During laser lithotripsy, the energy pulses form the laser fibre fragment the stones in very small pieces and can also create dust depending on the laser settings. The "cloud" created from the fragments and dust will obstructs the vision of the endoscope. Typically, an irrigation system is used to flush out the fragments and dust and clear the vision. Most commonly there is a constant low irrigation flow and the user can suddenly create peak of higher flow to rapidly flush the dust out and clear the vision faster.

There are many different irrigation systems and regulation methods worldwide, they can be made of bags with gravity or pressurized systems, regulated with hand pumps, syringes, pedals or the like, and all the practitioners' use them differently creating high dispersions in the way the irrigation is controlled during the procedure. The narrow tracks of the urinary system are usually filled with the required devices for the procedure such as the guide wire, an access sheath and a flexible endoscope or the like. This density of devices leaves very little room for the irrigation liquid to flow out and peaks of the irrigation flow will most likely create high peaks of pressure inside the kidney. As there is no control at all on the pressure generated, high intra-renal pressure generations are most likely to happen. Studies have shown that the pressure threshold of 30-40mmHg at which reflux occurs and the patient starts to be exposed to risks is almost always overpassed by all irrigation systems and users. Very high intrarenal pressure that could cause kidney injuries can even be generated.

Therefore, the state of the art, in particular the state of the art mentioned above, describes sensors that are incorporated into guide wires for monitoring the current pressure and temperature in the treatment zone or its environment / periphery. During laser lithotripsy, the heat generated by the laser can also be a concern and recent studies have shown that the irrigation helps keeping the temperature of the fluids at an acceptable level.

US 2009 0192412 A1 discloses a flexible guidewire comprising a hollow tube, having a proximal section and a distal section, the distal section having a distal tip, the outer diameter of the distal section gradually decreasing toward the distal tip, the outer diameter of the distal tip being larger than the smallest outer diameter of the distal section, the flexible guidewire further comprising a plug coupled with the distal tip of the hollow tube for creating a non-traumatic tip, and the flexible guidewire further comprising a tubular spring, being place around the distal section of the hollow tube for maintaining the outer diameter of the hollow tube over the length thereof and for supporting compressive loads.

US 2018 / 0 093 078 A1 discloses a guidewires having conductive elements are described where in one variation, the guidewire may be formed by disposing an insulative layer upon a surface of the guidewire core, and printing one or more conductive traces directly upon a surface of the insulative layer.

Considering the above, it was an object of the present invention to provide a guide wire being improved compared to the state of the art, in particular in view of a lifetime of the guide wire.

This object is achieved by the guide wire according to claim 1, a kit according to claim 14 a monitoring system according to claim 15. Preferred embodiments are incorporated in the dependent claims, the description and the figures.

According to the invention, a guide wire for endoscopic treatments, in particular for use in endourological interventions, is provided, comprising
- a proximal section and
- a distal section,
wherein a core is arranged inside the guide wire, wherein the core tapers from the proximal section to the distal section and is preferably made from a material being super elastic, such as for example Nitinol.

In contrast to the state of the art, the external extension of the core ensures the stiffness of the guide wire. Preferably, the conical tapper diameter reduction on the distal part ensures the flexibility and floppy aspect of the tip to not damage any tissue during its insertion. For example, the tip, extending preferably the last 8, more preferably the last 5 and most preferably the last 3 cm of the distal end, has an external extension smaller than 0.5 mm, more preferably smaller than 0.3 mm and most preferably smaller than 0.23 mm.

In particular, the distal section includes a particularly elastic and flexible element in the form of a spring element and a distal tip forming an end of the guide wire, wherein a sensor, in particular a pressure sensor and/or temperature sensor, is arranged between the spring element and the distal tip. Preferably it is provided that on top of the core comes, in a coaxial manner, a "jacket" that covers the whole body from proximal to distal. This "jacket" is preferably made of a metallic spring coil that has particularly a flat cross section. This "jacket" could also be made of polyurethane and/or a PTFE extrusion.

According to the present invention it is provided that the sensor is arranged between the spring element and the distal tip. Thus, the sensor is automatically placed close to the treatment zone or its environment / periphery.

Furthermore, incorporating the sensor has the positive effect of introducing no additional device to the treatment. Instead, the sensor is incorporated into a device that is used in an endoscopic treatment anyways, in particular every lithotripsy procedure, and is considered as standard in the treatment, namely a safety guide wire. Preferably, the guide wire is configured such that the specifications corresponds to standards used for guide wires in endoscopic treatments, in particular in urology.

Preferably, the spring element extends from the proximal section to the distal section and represents the longest part of the guide wire along the longitudinal direction of the guide wire. In particular, the spring element is a spring coil forming a sleeve like jacket of the guide wire. The distal tip forms the end of the guide wire at its distal end. Preferably, the distal tip is formed by a sleeve-like base body having a dome like structure at its end in longitudinal direction. In particular, it is provided that the spring element and the distal tip are flexible and elastic. It is also conceivable that a stiffness, in particular a bending stiffness, assigned to the distal tip is larger than a stiffness, in particular a bending stiffness, assigned to the spring element, preferably at least 5 times greater, more preferably at least 10 times greater or even at least 20 times greater. Furthermore, it is provided that the distal tip is made from a material different to the material of the spring element. For example, the spring element is metallic and the distal tip is made of polymer material.

Preferably, the sensor is a pressure sensor, such as a semiconductor (e.g., silicon wafer) pressure sensor, piezoelectric pressure sensor, a fiber optic or optical pressure sensor, a Fabry-Perot type pressure sensor, an ultrasound transducer and/or ultrasound pressure sensor, a magnetic pressure sensor and/or a solid-state pressure sensor. In particular, it is provided that the pressure sensor is adapted for measuring a relative pressure and preferably simultaneously the temperature. By using the intrinsic properties of the pressure sensor that due to its small size is highly sensitive to temperature changes one can deduce the temperature value and thus use the same sensor to both measure the pressure and the temperature. The sensor is, for example, an absolute pressure sensor being configured for performing a zeroing step at the beginning of the procedure before measuring the pressure in the kidney. As a consequence, it is possible that the pressure measured is relative.

According to a preferred embodiment of the present invention, it is provided that the sensor is arranged directly adjacent to the distal tip. Thus, the sensor, in particular a sensitive region of the sensor, follows the orientation of the distal tip. In particular, it is provided that the sensor is connected to the adjacent distal tip such that the relative orientation between the sensor and the distal tip is constant in operation, i. e. there is no relative movement such as rotation or shifting between the distal tip and the sensor.

In particular, the sensor is encapsulated in a housing, wherein preferably a first front side of the housing is connected to the distal tip and a second front side being opposite to the first front side is connected to the spring element. Thus, the housing of the sensor forms a transition between the distal tip and the spring element. For example, the housing is connected to the spring element and/or the distal tip in a form fitting manner, a force fitting manner and/or adhesively. It is conceivable that the housing is covered at least partially by the distal tip and/or the spring element. In other words: the housings extends along the longitudinal direction into the sleeve like body of the spring element and/or the sleeve like body of the distal tip. In particular, it is provided that the sensor is embedded into the housing in order to be hold securely by the housing. For example, the sensor is surrounded by a mould forming the housing, in particular a massive housing. Preferably, the housing forms a part of the outer side of the guide wire and the sensor is arranged inside the housings such that the sensitive region of the sensor forms a part of the outer side of the guide wire.

Preferably, it is provided that a distance between the sensor and the end of the guide wire at the distal section is between 2 and 5 cm, preferably 2.5 and 4.5 and most preferably about 3 cm. Thus, the length of the distal tip is shorter compared to those known in the state of the art that usually have a length of at least 5 cm. As a consequence, the sensor is automatically located close to the treatment zone.

Furthermore, it is preferably provided that the guide wire, in particular the spring element, and/or the housing, comprises a coating, in particular a PTFE coating and/or a hydrophilic coating. In particular, the distal tip has not such a coating. On the last few 8 cm, preferably the last 5 cm and most preferably the last 3 cm of the distal part, the core, in particular the core, is covered with polyurethane that has a radio component and/or a hydrophilic coating to facilitate its insertion through the urinary tracks. Preferably, the spring element is coated with a PTFE coating and the distal tip end is coated with a hydrophilic coating. Moreover, the distal tip comprises a radio component, to ensure opacity to radio waves and/or X-ray waves.

The distal part of the Nitinol core is preferably such thin that it is hardly visible under x-rays. When inserting the guide wire, the surgeon is looking at the x-ray to see the progression of the guide wire through the urinary tracks until it is safely positioned inside a calyx of the kidney, for example. Thus, the surgeon needs to have a clear vision of the distal tip under the x-rays. To ensure this, the polyurethane that covers the distal part is doped with a radio component that makes it visible under x-rays.

According to another preferred embodiment it is provided that the proximal section has a connection region for connecting the guide wire to an adapter.

A hand piece of the adapter preferably serves as a connector to which the guide wire is plugged into. The guide wire is plugged into the adapter to transmit the sensor signal after is has been safely positioned. The connection region allows connecting the guide wire reversible, to the hand piece. For instance, the connection region of the guide wire is configured jack-like. The jack-like design of the connector advantageously ensures a constant external diameter on the entire length of the guide wire. Moreover, it is provided that the adapter and the connection region are configured according to a key - lock principle.

Preferably, the guide wire, in particular in its proximal sections, has a diameter between 0.5 and 1.55 mm, preferably between 0.8 and 1.0 mm and most preferably mainly of 0.9 mm. Thus, the guide wire is dimensioned thin enough for keeping traumatization to tissues as low as possible.

In another preferred embodiment of the present invention, it is provided that a sensitive area of the sensor forms a part of the outer surface of the guide wire. Preferably, the sensor is embedded in the housing such that the sensitive region of the sensor and the outer side of the housing and/or guide wire forms a closed surface, in particular a complete closed and flat surface, i. a. surface without recesses.

Preferably, the sensor has a membrane insulation. Thus, it is possible to protect the sensor. Alternatively or additionally the sensor is made of a membrane and the whole sensor is covered and insulated by a gel or a soft biocompatible silicone that protects it and allow the pressure sensing. So it can be introduced in liquids.

It is also conceivable that the sensor, in particular the housing including the sensor, has wire bondings and / or wire connection insulation for connecting the sensor to a cable that extend from the sensor to the proximal end in order to provide in the connection region of the guide wire an output of the sensor. Furthermore, it is preferably provided that electrical wires are bond to the sensor and these electrical wires extend from the sensor to the jack-like connector of the proximal part to transmit the sensor signal. The bonds were the electrical wires are connected to the sensor are protected and insulated with a hard resine (epoxy like). The electrical wires are three wires that are parallel and attached together. They are wound around the nitinol core in a flat manner to reduce the space used between the Nitinol and the spring.

Furthermore, it is provided that the distal tip is made from plastic, in particular from a polymer. Preferably, the distal tip is made from polyurethane (PU).

For example, the core is made from a nickel-titanium alloy such as linear-elastic and/or super-elastic Nitinol, nickel alloys such as nickel-chromium-molybdenum alloys (e.g., UNS: N06625 such as INCONEL^{®} 625, UNS: N06022 such as HASTELLOY^{®} UNS: N10276 such as HASTELLOY^{®} C276^{®}, HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), nickel-chromium alloys, other nickel-molybdenum alloys, nickel-cobalt alloys, nickel-iron alloys, nickel-copper alloys, nickel-tungsten or tungsten alloys, cobalt-chromium alloys; cobalt-chromium-molybdenum alloys (e.g., UNS: R30003 such as ELGILOY^{®}, PHYNOX^{®}, and the like) and/or platinum enriched stainless steel or titanium.

Preferably, it is provided that in the guide wire the core has an extension measured in a direction perpendicular to the longitudinal direction of 0.5 to 0.6 mm, preferably of 0.54 to 0.56 mm and more preferably about 0.54 mm and/or in the distal section core has an extension measured in a direction perpendicular to the longitudinal direction of 0.3 to 0.1 mm, preferably of 0.17 and 0.22 mm and more preferably about 0.20 or 0.19 mm, in particular on the last 3 cm of the distal end. Between the proximal section and the distal section the core and preferably the whole distal part progressively tapers, wherein a region along that the core tapers has a length between 120 and 150 mm, preferably between 130 and 140 mm and more preferably about 135 mm.

Furthermore it is provided that the core extends through the housing and the core has its smallest extensions (measured in a direction perpendicular to the longitudinal direction) in the region extending through the housing. As a consequence, the size and the arrangement of the sensor and its contacts can be arranged flexible since the size of the core does not essential limit the space for arranging the sensor in the housing. Furthermore, it is provided that the housing has a recess or a hole for providing a channel and the core extends through that channel. Preferably, it is provided that a ratio of an extension (measured in a direction perpendicular to the longitudinal direction) of the hole of the housing to the extension (measured in a direction perpendicular to the longitudinal direction) of the core inside the hole is about 0.1 and 0.4 mm, preferably between 0.15 and 0.3 mm and more preferably between 0.18 and 0.22 mm.

In another preferred embodiment it is provided that a wire, preferably three wires, being connected to the sensor is wrapped around the core.. As a consequence the movement of the wire inside the guide wire is limited and a rearrangement of the wire inside the guide wire does not affect the functionality of the guide wire. The three electrical wires are attached particularly in a parallel manner which allows to used less space, when wound "flat" around the nitinol core instead of twisted and this allows for a higher external diameter for the core which, provides more stiffness and makes a better guide wire in term of ease of insertion.

Another aspect of the present invention is a kit comprising a guide wire according to the present invention and an adapter for connecting the guide wire to a human machine interface, such as a monitoring system, wherein the kit is preferably sterilized, preferably by ethylene oxide. For example the kit is provided as a packaged sterilized instrument / kit. Preferably, the adapter comprises a hand piece and a cable. The human machine interface might include a display and/or a processor for evaluating the data being provided by the sensor.

Another aspect of the present invention is a monitoring system comprising a human machine interface and the kit according to the present invention. In particular, the human machine interface is a display or a warning system that inform the user, when a critical threshold, in particular of the pressure, is exceeded. In particular, the human machine interface displays the temperature and pressure information as informative data and no thresholds for risk should be indicated. Preferably, the adapter is connected to the machine interface and the guide wire to the adapter in an operation state. In particular, the human machine interface, the adapter and the guide wire are configured such that a signal detected by the sensor is transmitted from the sensor to the human machine interface, for example via a radio signal and/or via a cable. Preferably, the signal is transferred from the sensor to the adapter and, in turn, from the adapter to the human machine interface.

For example, the sensor detects an intra-renal pressure exceeding the critical threshold value and an irrigation system is adapted automatically and/or the detected intra-renal pressure determines a post-operative monitoring of the patient.

Furthermore, it is provided that on top of the nitinol core is slipped the sensor housing. The sensor is preferably glued inside the sensor housing. The electrical wires are connected and wound around the nitinol core. The sensor bounds are protected and/or the sensor membrane is insulated. On the distal side of the sensor housing the nitinol is covered with a polyurethane jacket hat has a radio opaque component and a hydrophilic coated. On the other side of the sensor housing the nitinol and the electrical wires are covered with the spring element that acts as "jacket" and is particularly coated with PTFE. On the proximal part of the guide wire the spring element is attached to a jack-like connector that has the same external diameter. The jack like connector is made of three metallic ring to which the three electrical wires attached to the sensor are connected.

Wherever not already described explicitly, individual embodiments or their individual aspects and features can be combined or exchanged with one another without limiting or widening the scope of the described invention, whenever such a combination or exchange is meaningful and in the sense of this invention. Advantages which are described with respect to one embodiment of the present invention are, wherever applicable, also advantageous of other embodiments of the present invention.

In the drawings:
- Fig. 1a to 1c: schematically shows a monitoring system for endoscopic treatments
- Fig.2: schematically shows a guide wire according to a preferred embodiment of the present invention

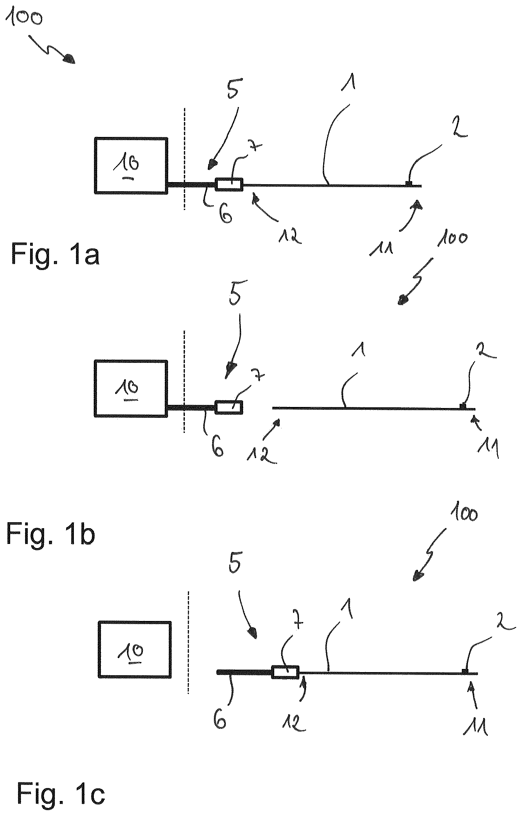

In the **Figures 1a to 1c** a monitoring system 100 for endoscopic treatments is schematically shown. In general, an endoscopic treatment, such as a treatment comprising flexible ureterorenoscopy and laser lithotripsy, is used for gaining visual insight into a body of a patient, in particular by using an endoscopic system introduced into the body of the patient. In particular, the endoscopic system comprises a guide wire 1. For enabling a good view a rinsing stream formed by a liquid is used to flush out faster debris generated by the fragmentation for clearing the vision. Said rinsing steam might be provided by an irrigation system via the endoscopic system or a separate rinsing channel via a separate endoscope. High rinsing streams, e.g., short, high-pressure ones for flushing the treatment zone, thus quickly trigger a significant pressure increase inside of a body. However, this leads to a risk of an intra-renal pressure resulting for example in intra-renal reflux, kidney injuries, fornix rupture, bleeding, hematoma, pain, infection, sepsis, postoperative complications or fever.

Therefore, there is an increased interest to know a current pressure at the treatment zone for adjusting the pressure in order to avoid complications. The monitoring system 100 presented in figure 1 is intended to provide the user information regarding an environment or periphery of the treatment zone, such as the pressure and/or temperature, by a human machine interface 10. The human machine interface 10 might be a monitor or an alarming system, for example for providing optical and/or acoustical warn signals to the user, such that the warning signal informs the user of the endoscope about exceeding a critical value of pressure.

For measuring the information, in particular the pressure, a sensor 2 is incorporated into a distal section 11 of a guide wire 1 of the endoscopic system, wherein the distal section 11 is directed to the treatment zone of the body in operation. Such a guide wire 1 might be intended for being advanced through a tube-like anatomic structure, such as a blood vessel or a body lumen, to the treatment zone. Since the guide wire 1 needs to pass tortuous anatomy, the guide wire 1 needs to be at least partially flexible and elastic. Besides the distal section 11 the guide wire 1 comprises a proximal section 12. In operation, the proximal section 12 is connected to an adapter 5 for connecting the guide wire 1 to a human machine interface 10. In an operation state the adapter 5 is in turn connect to the human machine interface 10 (see figure 1a). Preferably, the guide wire 1, in particular its proximal section 12, can be plugged into the adapter 5 by a corresponding plug-in mechanism. For example, it is provided for the plug-in mechanism that the proximal section 12 forms a male connection region 31, in particular a "Jack-like" connection region 31. By using the plug-in mechanism it is possible to connect the adapter 5 to the guide wire 1 and disconnect the adapter 5 from the guide wire 1, easily (see figure 1b).

Preferably, the adapter 5 comprises a hand piece 7 for controlling the movement of the guide wire 1 in operation and/or a cable 6, such as a 2.5 m long cable 6, for connecting the adapter 5 to the human machine interface 10. In particular, it is provided that the adapter 5 and the guide wire 1 form a kit that is provided in a sterilized way. The kit can be connected to the non-sterile human machine interface 10. In particular, the guide wire 1 and the adapter 5 forms a kit that can be connected to the human machine interface 10 as a removable part (see figure 1c).

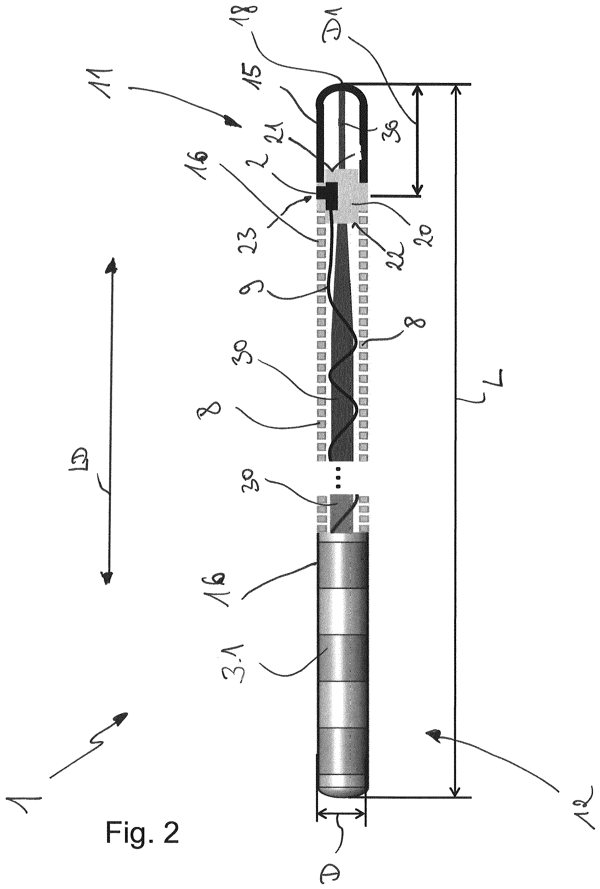

In **Figure 2** the guide wire 1 of Figure 1 is shown in detail. The guide wire 1 has a length L of 1500 mm and comprises a mainly cylinder-like sleeve 16 having an outer diameter D of 0.90 mm. In the proximal section 12 the guide wire 1 has the connection region 31. For example, the proximal section 12, in particular the connection region 31 of the proximal section 12, is made from plastic or stainless steel. Furthermore, it is provided that the guide wire 1 comprises a spring element 8. Preferably, the spring element 8 is realized as a coil spring that forms a part of the sleeve 16 of the guide wire 1. The distal section 12 includes, besides the spring element 8, a distal tip 15 that is preferably dome-shaped and forms the end 18 of the guide wire 1.

The distal tip 15, in particular the "jacket-like" distal tip 15, is preferably made from a plastic material, such as a polyurethane (PU), and in particular comprises a radio component and/or a hydrophilic coating. Especially, it is provided that the sensor 2 is arranged between the spring element 8 and the distal tip 15, in particular in a longitudinal direction LD of the guide wire 1. In particular, the sensor 2 is arranged directly next or adjacent to the distal tip 15. For example, the sensor 2 is connected directly to the distal tip 15 in a form-fitting, force-fitting and/or adhesive way. Furthermore, it is provided that the sensor 2 is encapsulated in a housing 20.

Thereby the housing 20 forms a transition from the distal tip 15 to the spring element 8. Preferably, the housing 20 is connected to the distal tip 15 at a first side 21 and is connect to the spring element 8 at a second side 22 being opposite to the first side 21 along the longitudinal direction LD of the guide wire 1. Furthermore, it is provided that a distance D1 from a distal end 18 of the guide wire 1 to the sensor 2 is about 2 to 5 cm, preferably 2.5 to 4.5 and most preferably about 3 cm. Further, it is provided that the housing 20 comprises besides the sensor 2 a wire bonding, a wire connection insulation and/or a sensor membrane insulation. Preferably, the sensor 2 is arranged such that a sensitive region 23 of the sensor 2 forms a part of the outer side of the guide wire 1, i. e. the sensitive region 23 of the sensor 2 is flushed with the outer side of the guide wire 1, in particular with the outer side of the spring element 8 and/or the distal tip 15.

As a consequence of the mentioned arrangement, the sensor 2 can be automatically arranged comparably close to the treatment zone in operation. In addition, by arranging the sensor 2 in the housing 20 and next to the distal tip 15, it is possible to protect the sensor 2.

Preferably the guide wire 1, or at least the distal tip 15, the housing 20 and/or the spring element 8 are covered at least partially, preferably completely by a coating. The coating may be made from a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Furthermore, it is provided that the guide wire 1 comprises a core 30 being arranged inside the sleeve 16 of the guide wire 1, in particular concentrically. Especially, the core 30 tapers along a longitudinal direction LD of the guide wire 1 from the proximal section 12 to the distal section 11. Preferably, the core 30 is made from a linear elastic and/or a super-elastic material such as nitinol. Further, a wire 9, preferably three wires, being connected to the sensor 2 is wrapped around the core 30.

Reference list:
- 1: guide wire
- 2: sensor
- 5: adapter
- 6: cable
- 7: hand piece
- 8: spring element
- 9: wire
- 10: human machine interface
- 11: distal section
- 12: proximal section
- 15: distal tip
- 16: sleeve
- 18: end
- 20: housing
- 21: first side
- 22: second side
- 23: sensitive region
- 30: core
- 31: connection region
- 100: monitoring system
- D: diameter
- D1: distance
- L: length
- LD: longitudinal direction

## Claims

1. Guide wire (1) for endoscopic treatments, in particular for use in endourological interventions, comprising
a sensor (2),
a proximal section (12) and
a distal section (11),
wherein a core (30) is inside the guide wire (1) and wherein the core (30) tapers from the proximal section (12) to the distal section (11) **characterized in that** said guide wire (11) includes a spring element (8) and a distal tip (15) forming an end (18) of the guide wire (1), wherein the sensor (2), in particular a pressure sensor and/or temperature sensor, is arranged between the spring element (8) and the distal tip (15).

2. Guide wire (1) according to claim 1, wherein the core (30) is made from a material being super elastic.

3. The guide wire (1) according to one of the preceding claims, wherein the sensor (2) is arranged directly adjacent to the distal tip (15).

4. The guide wire (1) according to one of the preceding claims, wherein the sensor (2) is encapsulated in a housing (20), wherein preferably a first side (21) of the housing (20) is connected to or part of the distal tip (15) and a second side (22) being opposite to the first side (21) is connected to the spring element (8).

5. The guide wire (1) according to one of the preceding claims, wherein a distance (D1) between the sensor (2) and the end (18) of the guide wire (1) at the distal section (11) is between 2 and 5 cm, preferably 2.5 and 4.5 cm and most preferably about 3 cm.

6. The guide wire (1) according to one of the preceding claims, wherein the guide wire (1), in particular the spring element (8) or the housing (20) according to claim 4, comprises a coating, in particular a PTFE coating and/or a hydrophilic coating.

7. The guide wire (1) according to one of the preceding claims, wherein the proximal section (12) has a connection region (31) for connecting the guide wire (1) to an adapter (5).

8. The guide wire (1) according to one of the preceding claims, wherein the guide wire (1), in particular in its proximal section (12), has a diameter (D) between 0.5 and 1.55 mm, preferably between 0.8 and 1.0 mm and most preferably of mainly 0.9 mm.

9. The guide wire (1) according to one of the preceding claims, wherein a sensitive area (23) of the sensor (2) forms a part of the outer surface of the guide wire (1).

10. The guide wire (1) according to one of the preceding claims, wherein the distal tip (15) is made from plastic, in particular from a polymer.

11. The guide wire (1) according to one of the preceding claims, wherein the core (30) made from a material being super elastic is Nitinol.

12. The guide wire (1) according to claims 4 and 11 , wherein the core (30) extends through the housing (20) and preferably the core (30) has its smallest extensions in the region extending through the housing (20).

13. The guide wire (1) according to claim 11 or 12, wherein at least one wire (9), preferably three wires of electrical wires, being connected to the sensor (2) is wrapped around the core (30).

14. Kit comprising a guide wire (1) according to one of the preceding claims and an adapter (5) for connecting the guide wire (1) to a human machine interface (10), wherein the kit is preferably sterilized.

15. Monitoring system (100) comprising a human machine interface (10) and the kit according to claim 14.

## Patentansprüche

1. Führungsdraht (1) für endoskopische Behandlungen, insbesondere zur Verwendung bei endourologischen Eingriffen, umfassend
einen Sensor (2),
einen proximalen Abschnitt (12) und
einen distalen Abschnitt (11),
wobei sich ein Kern (30) im Inneren des Führungsdrahtes (1) befindet und wobei sich der Kern (30) vom proximalen Abschnitt (12) zum distalen Abschnitt (11) verjüngt, **dadurch gekennzeichnet, dass** der Führungsdraht (11) ein Federelement (8) und eine distale Spitze (15) einschließt, die ein Ende (18) des Führungsdrahtes (1) bildet, wobei der Sensor (2), insbesondere ein Drucksensor und/oder Temperatursensor, zwischen dem Federelement (8) und der distalen Spitze (15) angeordnet ist.

2. Führungsdraht (1) nach Anspruch 1, wobei der Kern (30) aus einem Material hergestellt ist, das superelastisch ist.

3. Führungsdraht (1) nach einem der vorangehenden Ansprüche, wobei der Sensor (2) unmittelbar neben der distalen Spitze (15) angeordnet ist.

4. Führungsdraht (1) nach einem der vorangehenden Ansprüche, wobei der Sensor (2) in einem Gehäuse (20) verkapselt ist, wobei vorzugsweise eine erste Seite (21) des Gehäuses (20) mit der distalen Spitze (15) verbunden oder ein Teil davon ist und eine zweite, der ersten Seite (21) gegenüberliegende Seite (22) mit dem Federelement (8) verbunden ist.

5. Führungsdraht (1) nach einem der vorangehenden Ansprüche, wobei ein Abstand (D1) zwischen dem Sensor (2) und dem Ende (18) des Führungsdrahtes (1) am distalen Abschnitt (11) zwischen 2 und 5 cm, vorzugsweise 2,5 und 4,5 cm und am meisten bevorzugt etwa 3 cm beträgt.

6. Führungsdraht (1) nach einem der vorangehenden Ansprüche, wobei der Führungsdraht (1), insbesondere das Federelement (8) oder das Gehäuse (20) nach Anspruch 4, eine Beschichtung, insbesondere eine PTFE-Beschichtung und/oder eine hydrophile Beschichtung, umfasst.

7. Führungsdraht (1) nach einem der vorangehenden Ansprüche, wobei der proximale Abschnitt (12) eine Verbindungsregion (31) zum Verbinden des Führungsdrahtes (1) mit einem Adapter (5) aufweist.

8. Führungsdraht (1) nach einem der vorangehenden Ansprüche, wobei der Führungsdraht (1), insbesondere in seinem proximalen Abschnitt (12), einen Durchmesser (D) zwischen 0,5 und 1,55 mm, vorzugsweise zwischen 0,8 und 1,0 mm und am meisten bevorzugt von hauptsächlich 0,9 mm aufweist.

9. Führungsdraht (1) nach einem der vorangehenden Ansprüche, wobei ein sensibler Bereich (23) des Sensors (2) einen Teil der Außenfläche des Führungsdrahtes (1) bildet.

10. Führungsdraht (1) nach einem der vorangehenden Ansprüche, wobei die distale Spitze (15) aus Kunststoff, insbesondere aus einem Polymer, hergestellt ist.

11. Führungsdraht (1) nach einem der vorangehenden Ansprüche, wobei der Kern (30) aus einem superelastischen Material wie Nitinol besteht.

12. Führungsdraht (1) nach Anspruch 4 und 11, wobei sich der Kern (30) durch das Gehäuse (20) hindurch erstreckt und der Kern (30) vorzugsweise in der Region, die sich durch das Gehäuse (20) hindurch erstreckt, seine kleinste Ausdehnungen aufweist.

13. Führungsdraht (1) nach Anspruch 11 oder 12, wobei mindestens ein Draht (9), vorzugsweise drei Drähte von elektrischen Drähten, die mit dem Sensor (2) verbunden sind, um den Kern (30) gewickelt sind.

14. Kit umfassend einen Führungsdraht (1) nach einem der vorangehenden Ansprüche und einen Adapter (5) zum Verbinden des Führungsdrahtes (1) mit einer menschlichen Maschinenschnittstelle (10), wobei das Kit vorzugsweise sterilisiert ist.

15. Überwachungssystem (100), umfassend eine Mensch-Maschine-Schnittstelle (10) und das Kit nach Anspruch 14.

## Revendications

1. Fil de guidage (1) pour les traitements endoscopiques, en particulier pour l'utilisation dans des interventions endo-urologiques, comprenant
un capteur (2),
une section proximale (12) et
une section distale (11),
dans lequel une âme (30) se trouve à l'intérieur du fil de guidage (1) et l'âme (30) s'effile de la section proximale (12) vers la section distale (11),
**caractérisé en ce que**
ledit fil de guidage (11) comprend un élément de ressort (8) et une pointe distale (15) formant une extrémité (18) du fil de guidage (1), le capteur (2), en particulier un capteur de pression et/ou un capteur de température, étant disposé entre l'élément de ressort (8) et la pointe distale (15).

2. Fil de guidage (1) selon la revendication 1, dans lequel l'âme (30) est constituée d'un matériau super-élastique.

3. Fil de guidage (1) selon l'une des revendications précédentes, dans lequel le capteur (2) est disposé de manière directement adjacente à la pointe distale (15).

4. Fil de guidage (1) selon l'une des revendications précédentes, dans lequel le capteur (2) est encapsulé dans un boîtier (20), de préférence un premier côté (21) du boîtier (20) étant relié à ou faisant partie de la pointe distale (15), et un deuxième côté (22) opposé au premier côté (21) étant relié à l'élément de ressort (8).

5. Fil de guidage (1) selon l'une des revendications précédentes, dans lequel une distance (D1) entre le capteur (2) et l'extrémité (18) du fil de guidage (1) au niveau de la section distale (11) est comprise entre 2 et 5 cm, de préférence entre 2,5 et 4,5 cm, et est plus préférablement d'environ 3 cm.

6. Fil de guidage (1) selon l'une des revendications précédentes, dans lequel le fil de guidage (1), en particulier l'élément de ressort (8) ou le boîtier (20) selon la revendication 4 comprend un revêtement, en particulier un revêtement PTFE et/ou un revêtement hydrophile.

7. Fil de guidage (1) selon l'une des revendications précédentes, dans lequel la section proximale (12) présente une zone de liaison (31) pour relier le fil de guidage (1) à un adaptateur (5).

8. Fil de guidage (1) selon l'une des revendications précédentes, dans lequel le fil de guidage (1), en particulier dans sa section proximale (12), présente un diamètre (D) compris entre 0,5 et 1,55 mm, de préférence entre 0,8 et 1,0 mm, et plus préférablement sensiblement de 0,9 mm.

9. Fil de guidage (1) selon l'une des revendications précédentes, dans lequel une zone sensible (23) du capteur (2) forme une partie de la surface extérieure du fil de guidage (1).

10. Fil de guidage (1) selon l'une des revendications précédentes, dans lequel la pointe distale (15) est réalisée en matière plastique, en particulier en polymère.

11. Fil de guidage (1) selon l'une des revendications précédentes, dans lequel l'âme (30) réalisée en un matériau super-élastique consiste en Nitinol.

12. Fil de guidage (1) selon les revendications 4 et 11, dans lequel l'âme (30) s'étend à travers le boîtier (20), et de préférence l'âme (30) présente ses plus petites extensions dans la zone s'étendant à travers le boîtier (20).

13. Fil de guidage (1) selon la revendication 11 ou 12, dans lequel au moins un fil (9), de préférence trois fils constitués de fils électriques, relié(s) au capteur (2), est enroulé autour de l'âme (30).

14. Kit comprenant un fil de guidage (1) selon l'une des revendications précédentes et un adaptateur (5) pour relier le fil de guidage (1) à une interface homme-machine (10), dans lequel le kit est de préférence stérilisé.

15. Système de surveillance (100) comprenant une interface homme-machine (10) et le kit selon la revendication 14.
